(19) Europäisches Patentamt
European Patent Office
Office européen des brevets

(11) Veröffentlichungsnummer: **0 000 750**
**A1**

(12) # EUROPÄISCHE PATENTANMELDUNG

(21) Anmeldenummer: **78100536.8**

(22) Anmeldetag: **28.07.78**

(51) Int. Cl.$^2$: **C 07 D 275/04,** C 07 D 417/04 //C07D417/10,A61K31/505,A61K31/425

(30) Priorität: **03.08.77 DE 2734866**

(43) Veröffentlichungstag der Anmeldung: **21.02.79 Patentblatt 79/4**

(84) Benannte Vertragsstaaten: **BE CH DE FR GB NL**

(71) Anmelder: **BASF Aktiengesellschaft**
**Carl-Bosch-Strasse 38**
**D-6700 Ludwigshafen(DE)**

(72) Erfinder: **Hagen, Helmut, Dr.**
**Max-Slevogt-Strasse 17e**
**D-6710 Frankenthal(DE)**

(72) Erfinder: **Kohler, Rolf-Dieter, Dr.**
**Speyerer Strasse 3**
**D-6800 Mannheim 23(DE)**

(72) Erfinder: **Markert, Juergen, Dr.**
**Lisztstrasse 117**
**D-6700 Ludwigshafen(DE)**

(54) **Neue 1,2-Benzisothiazole und Verfahren zu ihrer Herstellung.**

(57) Neue 1,2-Benzisothiazole und ein Verfahren zur Herstellung von 1,2-Benzisothiazolen durch Umsetzung von o-Halogenarylketonen mit Ammoniak und elementarem Schwefel.

Die nach dem Verfahren der Erfindung herstellbaren Verbindungen sind wertvolle Ausgangsstoffe für die Herstellung von Farbstoffen, Pflanzenschutzmitteln und Pharmazeutika.

EP 0 000 750 A1

BASF Aktiengesellschaft O. Z. 0050/032721

Neue 1,2-Benzisothiazole und Verfahren zu ihrer Herstellung

Die Erfindung betrifft neue 1,2-Benzisothiazole und ein Verfahren zur Herstellung von 1,2-Benzisothiazolen durch Umsetzung von o-Halogenarylketonen mit Ammoniak und elementarem Schwefel.

Es ist aus der Angewandten Chemie, Band 36, Seite 159 (1923) und aus den Berichten der deutschen Chemischen Gesellschaft, Band 58, Seite 2 095 (1925) bekannt, Thionaphthen-2,3-dion mit Ammoniak und Wasserstoffperoxid zu 3-Carbamyl-1,2-benzisothiazol umzusetzen und daraus durch Hydrolyse und Decarboxylierung 1,2-Benzisothiazol zu gewinnen. Die vorgenannten Berichte, Band 56, Seite 1 630 (1923) und Liebigs Annalen der Chemie, Band 454, Seite 264 (1927) beschreiben die Umsetzung von 2-Formyl-4-nitrophenylsulfonenylbromid mit Ammoniak zu 5-Nitro-1,2-benzisothiazol. Ebenfalls kann man Benzisothiazole durch Cyclisierung von o-Mercapto-phenyl-carbonyl-verbindungen in Gegenwart von Polyphosphorsäure synthetisieren (Annali di Chimica, Band 53, Nummer 5, Seiten 577 bis 587 (1963)). Es ist aus der deutschen Offenlegungsschrift 1 670 196 bekannt, daß man Dihalogenmethylarylverbindungen mit Ammoniak und Schwefel zu Benzisothiazolen umsetzt. Alle diese Ver-

WB/IG

fahren sind mit Bezug auf leicht zugängliche Ausgangsstoffe, Wirtschaftlichkeit, Einfachheit des Betriebs bei gleichzeitig guter Ausbeute an Endstoff unbefriedigend.

Gegenstand der DOS 25 03 699 ist ein Verfahren zur Herstellung von 1,2-Benzisothiazolen der Formel

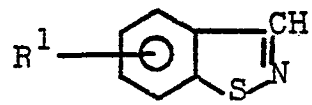 Ia,

in der $R^1$ Wasserstoff, einen aliphatischen, cycloaliphatischen oder einen, gegebenenfalls anellierten, aromatischen Rest, Halogen, eine Alkoxy- oder Nitrogruppe oder den Rest 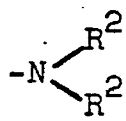, worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, wobei man o-Halogenarylaldehyde der Formel

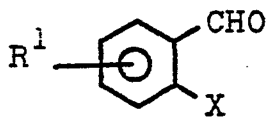 IIa,

in der X für Halogen steht und $R^1$ die vorgenannte Bedeutung hat, mit Ammoniak und elementarem Schwefel umsetzt.

Es wurde nun gefunden, daß man 1,2-Benzisothiazole der Formel

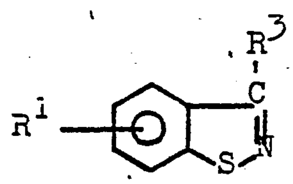 I,

worin $R^1$ Wasserstoff, einen aliphatischen, cycloaliphatischen,

tischen oder einen, gegebenenfalls anellierten, aromatischen Rest, Halogen, eine Alkoxygruppe, Nitrogruppe, oder den Rest

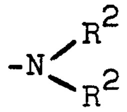 , worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, $R^3$ für einen aromatischen oder heterocyclischen Rest steht, vorteilhaft herstellt, wenn man o-Halogenarylketone der Formel

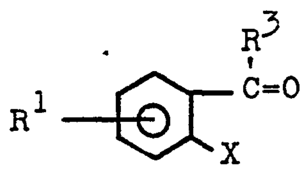 II,

worin $R^1$ und $R^3$ die vorgenannte Bedeutung besitzen und X für Halogen steht, mit Ammoniak und elementarem Schwefel umsetzt.

Weiterhin wurden die neuen 1,2-Benzisothiazole der Formel

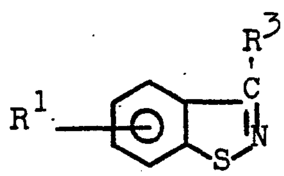 I,

worin $R^1$ Wasserstoff, einen aliphatischen, cycloaliphatischen oder einen, gegebenenfalls anellierten, aromatischen Rest, Halogen, eine Alkoxygruppe, Nitrogruppe oder den Rest

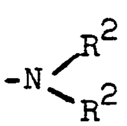 , worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, und $R^3$ für einen substituierten ein-

kernigen oder unsubstituierten mehrkernigen oder substituierten mehrkernigen, aromatischen Rest oder einen heterocyclischen Rest steht, gefunden.

Die Reaktion läßt sich am Beispiel der Umsetzung von 2-Chlor-5-nitrobenzophenon mit Ammoniak und Schwefel formelmäßig wie folgt wiedergeben:

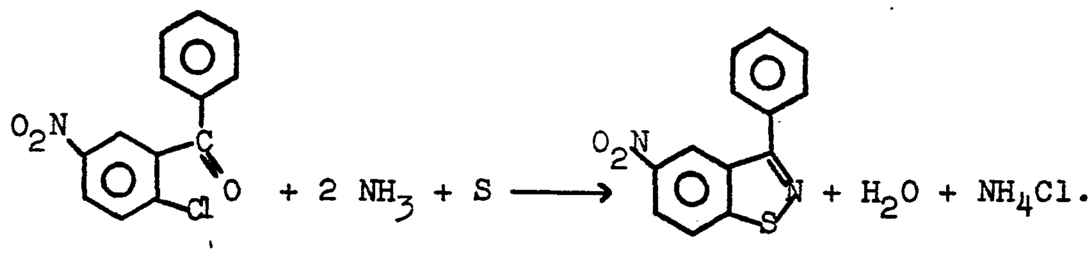


Im Vergleich zu den erstgenannten, bekannten Verfahren geht das Verfahren nach der Erfindung von leichter zugänglichen Ausgangsstoffen aus und liefert überraschend auf einfache und wirtschaftliche Weise 1,2-Benzisothiazole in besserer Ausbeute und Reinheit. Im Hinblick auf die deutsche Offenlegungsschrift 25 03 699 können auch die Ketone II zu bisher nicht beschriebenen, in 3-Stellung durch aromatische oder heterocyclische Reste substituierten 1,2-Benzisothiazolen umgesetzt werden. Diese vorteilhaften Ergebnisse sind im Hinblick auf den Stand der Technik überraschend.

Bevorzugte Ausgangsstoffe II und dementsprechend bevorzugte 1,2-Benzisothiazole I sind solche, in deren Formeln $R^1$ einen gegebenenfalls durch eine Carbonamidgruppe substituierten Alkylrest mit 1 bis 6 Kohlenstoffatomen, einen Cycloalkylrest mit 5 bis 12 Kohlenstoffatomen, einen Phenyl- oder Naphthylrest, die jeweils auch anelliert sein können, einen anellierten Naphthochinon-(1,4)-ylenrest, Wasserstoff, Brom oder insbesondere Chlor, eine Alkoxygruppe mit 1 bis 4 Kohlenstoffatomen, eine Nitrogruppe oder den Rest $-N{<}^{R^2}_{R^2}$,

worin die einzelnen Reste $R^2$ gleich oder verschieden sein

können und jeweils Wasserstoff, einen Alkylrest mit 1 bis 6 Kohlenstoffatomen, einen Alkenylrest mit 3 bis 6 Kohlenstoffatomen, einen Cycloalkylrest mit 5 bis 8 Kohlenstoffatomen, einen Aralkylrest mit 7 bis 12 Kohlenstoffatomen, einen Phenylrest bedeuten, bezeichnet, $R^3$ für einen unsubstituierten oder einen durch ein oder mehrere, vorzugsweise ein oder zwei, Alkylreste, vorzugsweise mit 1 bis 4 Kohlenstoffatomen, Alkoxygruppen, vorzugsweise mit 1 bis 4 Kohlenstoffatomen, Chloratome, Bromatome, Dialkylaminogruppen, vorzugsweise mit 1 bis 4 Kohlenstoffatomen je Alkylgruppe, substituierten Phenylrest oder Naphthylrest oder einen 5- oder 6-gliedrigen, heterocyclischen Ring, der zwei Stickstoffatome oder ein Stickstoffatom, ein Sauerstoffatom und/oder ein Schwefelatom enthalten kann, steht. Die vorgenannten Reste können noch durch unter den Reaktionsbedingungen inerte Gruppen, z.B. Alkylgruppen oder Alkoxygruppen mit jeweils 1 bis 4 Kohlenstoffatomen, Nitrogruppen, Carbonamidogruppen, substituiert sein. Statt der Ausgangsstoffe II können auch Stoffe, die die Ausgangsstoffe II unter den Reaktionsbedingungen bilden, z.B. die entsprechenden Aldehydacetale wie o-Chlorbenzophenon-dimethylacetal, verwendet werden. Ausgangsstoffe II, Ammoniak und elementarer Schwefel können in etwa stöchiometrischen Mengen verwendet werden, jedoch verwendet man vorzugsweise ein Verhältnis von 2 bis 10 Mol Ammoniak und/oder von 0,9 bis 1,1 Grammatom Schwefel je Mol Ausgangsstoff II.

Als Ausgangsstoffe II kommen beispielsweise in Betracht: 5-Nitro-, 4-Dimethylamino-, 4-Diäthylamino-, 4-Diallylamino-, 4-Di-(2'-methylallyl)-amino-, 4-(N-Methyl-N-2-carbonamido-äthylamino)-, 6-Methyl-, 3-Äthyl-, 5-Hexyl-, 6-Isobutyl-, 5-Propyl-, 4-tert.-Butyl-, 4-Cyclohexyl-, 4-Cyclopentyl-, 5-Phenyl-, 4-Phenyl-, 4-Nitrophenyl-, 4-p-Toluyl-, 4-p-Äthoxyphenyl-, 3-Di-(2'-äthoxyäthyl)-amino-, 4-Naphthyl-, 4-Brom-, 6-Methoxy-, 6-Dicyclohexylamino-,

4-Dibenzylamino-, 4-Diphenylamino-, 4-p-Xylyl-2-chlorbenzophenon; 2-Chlorbenzophenon, 2-Brombenzophenon; 1-Chlor-2-benzoyl-, 2-Chlor-3-benzoyl-, 2-Chlor-1-benzoyl-naphthalin; 1-Chlor-2-benzoyl-, 2-Chlor-3-benzoyl-, 2-Chlor-1-benzoyl-anthrachinon; 1-Chlor-2-benzoyl-, 2-Chlor-3-benzoyl-, 2-Chlor-1-benzoyl-anthrachinon; 1-Chlor-2-benzoyl-, 2-Chlor-3-benzoyl-, 2-Chlor-1-benzoyl-anthracen; 1-Chlor-2-benzoyl-, 2-Chlor-3-benzoyl-, 2-Chlor-1-benzoyl-phenanthren; entsprechend substituierte Bromarylketone; analog mit vorgenannten Substituenten an beiden Phenylkernen substituierte Benzophenone; analoge Aroylbenzole, deren Aroylrest mit Chlor oder Brom substituiert ist und die am Benzolkern in o-, m- und/oder p-Stellung ein oder zwei Methyl-, Äthyl-, Propyl-, Isopropyl-, Butyl-, Isobutyl-, sek.-Butyl-, tert.-Butyl-, Chlor-, Brom-, Äthoxy-, Methoxy-, Propoxy-, Isopropoxy-, Butoxy-, Dimethylamino-, Diäthylamino-, Dipropylamino-, Diisopropylamino-gruppen tragen; mit vorgenannten Substituenten am Aroylkern substituierte Aroylnaphthaline, Aroylthiophene, Aroyltetrahydrofurane, Aroylpyrane und entsprechende Aroylverbindungen von Imidazol, 1-Methylimidazol, 1-Propylimidazol, 2-Methylpyridin, 3-Methylpyridin, 4-Methylpyridin, 2,4-Dimethylpyridin, 2,6-Dimethylpyridin, 2,4,6-Trimethylpyridin, Pyridin, Pyrrolidin, Pyrrol, Imidazolidin, Piperidin, Morpholin, Pyridazin, Pyrimidin, Pyrazin, Piperazin.

Die Umsetzung wird in der Regel bei einer Temperatur von 20 bis 250°C, vorteilhaft von 20 bis 200°C, vorzugsweise von 40 bis 180°C, insbesondere von 40 bis 120°C, drucklos oder unter Druck, kontinuierlich oder diskontinuierlich durchgeführt. Der Reaktionsdruck wird im allgemeinen durch den Gesamtdampfdruck der Komponenten bei der Umsetzungstemperatur bedingt. Gegebenenfalls kann man unter den Reaktionsbedingungen inerte, organische Lösungsmittel verwenden, z.B. aromatische Kohlenwasserstoffe wie Toluol,

Äthylbenzol, o-, m-, p-Xylol, Isopropylbenzol; Alkanole und Cycloalkanole wie Äthanol, n-Butanol, Isobutanol, Methylglykol, Cyclohexanol, Propanol, Methanol, 2-Äthylhexanol; Äther, z.B. Äthylpropyläther, Diisobutyläther, Methyl-tert.-butyläther, n-Butyläthyläther, Di-n-butyläther, Dioxan, Diisoamyläther, Diisopropyläther, Anisol, Phenetol, Cyclohexylmethyläther, Diäthyläther, Tetrahydrofuran, Thioanisol; und entsprechende Gemische. Zweckmäßig verwendet man das Lösungsmittel in einer Menge von 200 bis 10 000 Gewichtsprozent, vorzugsweise von 300 bis 1 000 Gewichtsprozent, bezogen auf Ausgangsstoff II.

Die Reaktion kann wie folgt durchgeführt werden: Ausgangsstoff II, elementarer Schwefel und Ammoniak, gegebenenfalls in Gegenwart eines Lösungsmittels, werden in einem Druckreaktor während 3 bis 10 Stunden bei der vorgenannten Temperatur miteinander umgesetzt. Aus dem Reaktionsgemisch erhält man das 1,2-Benzisothiazol I nach den üblichen Verfahren, z.B. durch fraktionierte Destillation, Filtration und gegebenenfalls anschließender Umkristallisation aus einem geeigneten Lösungsmittel, z.B. Ligroin. Man kann das Reaktionsgemisch auch nach Entfernung überflüssigen Ammoniaks und Lösungsmittels in Wasser gießen, das gebildete Gemisch mit einem geeigneten Lösungsmittel, z.B. Methylenchlorid, Benzol, extrahieren und den Extrakt in vorgenannter Weise aufarbeiten.

Die nach dem Verfahren der Erfindung herstellbaren Verbindungen sind wertvolle Ausgangsstoffe für die Herstellung von Farbstoffe, Pflanzenschutzmitteln und Pharmazeutika.

Vorteilhaft sind für vorgenannte Verwendungen die 1,2-Benzisothiazole mit den bevorzugten Bedeutungen von $R^1$ und $R^3$ geeignet. So können z.B. aus 1,2-Benzisothiazolen der Formel

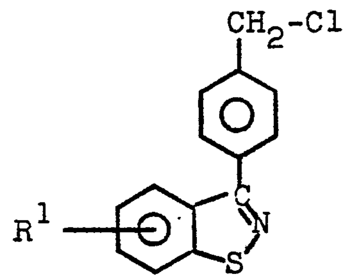

Ib,

in der $R^1$ die obengenannte Bedeutung hat, durch Umsetzung mit einem Diamin der allgemeinen Formel

$$H_2N - Y - NH_2 \qquad III,$$

in der Y ein Brückenglied der Formel

$$-CH_2-CH_2-, \quad -\underset{CH_3}{CH}-CH_2- \quad \text{oder} \quad -CH_2-CH_2-CH_2-$$

darstellt, und elementaren Schwefel, zweckmäßigerweise in einem inerten, organischen Lösungsmittel, Verbindungen der Formel

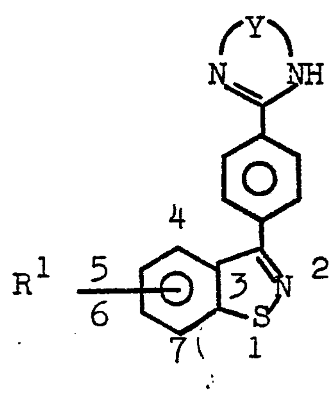

IV,

in der $R^1$ die vorgenannte Bedeutung, insbesondere ein Wasserstoffatom, einen Alkylrest mit 1 bis 4 C-Atomen, einen Alkoxyrest mit 1 bis 3 C-Atomen, ein Halogenatom oder eine Nitrogruppe bedeutet und Y die angegebene Bedeutung hat, hergestellt und gegebenenfalls die so erhaltenen Verbindungen IV in physiologisch verträgliche Säureadditionssalze übergeführt werden. Von den genannten

Bedeutungen sind als bevorzugt für $R^1$ ein Wasserstoffatom, ein Halogenatom, insbesondere ein Chloratom oder Bromatom, eine Nitrogruppe, einen Alkylrest oder einen Alkoxyrest mit jeweils 1 bis 4 C-Atomen und für Y 1-Methyläthylen-1,2 und Trimethylen-1,3 zu nennen. Als besonders bevorzugt sind für $R^1$ Wasserstoff und für Y 1-Methyläthylen-1,2 und Trimethylen-1,3 hervorzuheben.

Die vorgenannte Umsetzung der Verbindungen IV wird zweckmäßig bei Temperaturen von 40 bis 150°C, bevorzugt bei Temperaturen von 70 bis 120°C, durchgeführt.

Zweckmäßige Lösungsmittel für die Umsetzung der Verbindungen IV sind aromatische Kohlenwasserstoffe, insbesondere Benzolkohlenwasserstoffe, wie Benzol oder Toluol, niedere Alkohole, wie Methanol, Äthanol, Propanol, Isopropanol, Butanol oder Isobutanol, gesättigte cyclische oder aliphatische Äther, wie Dibutyläther oder Dioxan, Glykoläther, insbesondere Monoalkyläther des Glykols, wie Glykolmonomethyläther oder Glykolmonoäthyläther, oder Mischungen der genannten Lösungsmittel.

Von den genannten Lösungsmitteln sind die Benzolkohlenwasserstoffe, zweckmäßig Benzol und Toluol, und Monoalkyläther des Glykols, insbesondere Glykolmonomethyläther, bevorzugt.

Das Diamin der Formel III wird, berechnet auf die Verbindung der Formel I oder Ib, in stöchiometrischer oder in überschüssiger Menge, gegebenenfalls bis zur 3-fachen stöchiometrischen Menge, verwendet.

Der elementare Schwefel wird, berechnet auf die Verbindung Ib, in stöchiometrischer oder in überschüssiger Menge bis zur 1,2-fachen Menge über der stöchiometrischen Menge ver-

wendet. Bevorzugt wird die stöchiometrische Menge elementarer Schwefel eingesetzt.

Die Umsetzung von beispielsweise 3-(4-Chlormethylphenyl)-1,2-benzisothiazol mit Äthylendiamin und Schwefel kann durch die folgende Reaktionsgleichung beschrieben werden:

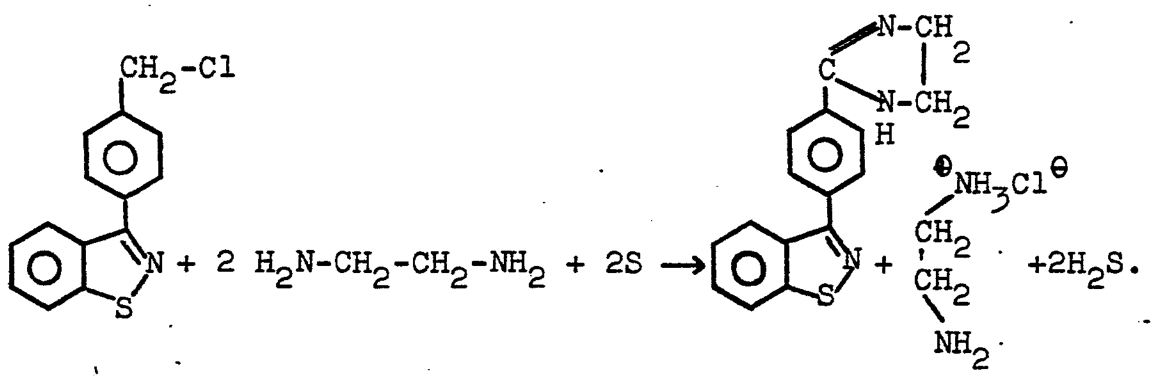

Die Ausgangsverbindungen der Formel Ib können beispielsweise durch Seitenkettenchlorierung von 3-(4-Methylphenyl)-1,2-benzisothiazolen I mit Chlor bei etwa 170°C und unter UV-Bestrahlung erhalten werden.

Die Verbindungen der allgemeinen Formel IV werden gegebenenfalls in an sich üblicher Weise in das Säureadditionssalz einer physiologisch verträglichen Säure überführt. Als übliche physiologisch verträgliche organische oder anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung, Band 10, Seiten 224 bis 225, Birkhäuser Verlag, Basel und Stuttgart, 1966, entnommen werden. Als vorteil-

hafte Verbindungen der Formel IV sind beispielsweise zu nennen:

3-[4-(Imidazolin-2-yl)-phenyl]-1,2-benzisothiazol

3-[4-(Methylimidazolin-2-yl)-phenyl]-1,2-benzisothiazol

3-[4-(Tetrahydropyrimidin-2-yl)-phenyl]-1,2-benzisothiazol

5-Chlor-3-[4-(imidazolin-2-yl)-phenyl]-1,2-benzisothiazol

5-Chlor-3-[4-(methylimidazolin-2-yl)-phenyl]-1,2-benzisothiazol

4-Methoxy-3-[4-(methylimidazolin-2-yl)-phenyl]-1,2-benzisothiazol

5-Nitro-3-[4-(imidazolin-2-yl)-phenyl]-1,2-benzisothiazol

5-Nitro-3-[4-(tetrahydropyrimidin-2-yl)-phenyl]-1,2-benzisothiazol

5-Brom-6-chlor-3-[4-(imidazolin-2-yl)-phenyl]-1,2-benzisothiazol.

Die vorgenannten 3-[4-(1,3-Diazacycloalken-2-yl)-phenyl]-1,2-benzisothiazole IV und ihre physiologisch verträglichen Säureadditionssalze weisen wertvolle pharmakologische Eigenschaften auf. Aus ihnen können pharmazeutische Zubereitungen, die neben üblichen pharmazeutischen Träger- und Verdünnungsmitteln eine Verbindung der Formel IV als Wirkstoff enthält, hergestellt werden. Besonders geeignet sind 3-[4-(Tetrahydropyrimidin-2-yl)-phenyl]-1,2-benzisothiazol, 3-[4-(Methylimidazolin-2-yl)-phenyl]-1,2-benzisothiazol und ihre physiologisch verträglichen Säureadditionssalze. Die Stoffe IV und ihre physiologisch verträglichen Säureadditionssalze zeichnen sich durch eine starke antiarrhythmische Wirkung aus und sind besonders zur Pharmakotherapie von Herzrhythmusstörungen geeignet. Zur Bestimmung ihrer antiarrhythmischen Wirksamkeit wurden die Substanzen Ratten (Stamm: Sprague Dawley, Gewicht: 180 - 240 g) 45 Minuten vor Beginn der Narkose (Thiobutabarbital 100 mg/kg i.p.) oral appliziert.

Als arrhythmogene Substanz diente Aconitin, das 60 Minuten nach der Substanzapplikation intravenös infundiert wurde (Dosierungsgeschwindigkeit: 0,005 mg/kg · Minute). Bei nichtbehandelten Tieren (N = 30) treten nach durchschnittlich 3,7 ± 0,9 Minuten Arrhythmien auf, deren Eintritt durch Antiarrhythmica dosisabhängig verzögert werden kann.

Zur quantitativen Auswertung der linearen Beziehung zwischen dem Logarithmus der Dosis (mg/kg) der Prüfsubstanzen und der relativen Verlängerung der Aconitininfusionsdauer (Δ %) wurde die Dosis bestimmt, welche die Infusionsdauer um 50 % verlängert (ED 50 %) Als Vergleichssubstanz diente das bekannte Antiarrhythmicum Procainamid.

Die akute Toxizität wurde an Gruppen von je 10 oder 20 weiblichen Swiss-Mäusen, Gewicht 20 - 26 g, bei intraperitonealer Applikation ermittelt. Als LD 50 wurde die Dosis berechnet (Probit-Analyse), nach der 50 % der Tiere innerhalb von 7 Tagen starben.

Die Tabelle 1 zeigt, daß die Verbindungen der Beispiel 2 und 3, verglichen mit dem Antiarrhythmicum Procainamid, rund 5 mal stärker antiarrhythmisch wirksam sind. Ein weiterer Vorteil besteht darin, daß die Wirkung der Maximaldosis um 114 (Beispiel 2) bzw. 73 % (Beispiel 3) höhere Werte erreicht, als die von Procainamid; d.h., daß der Aconitinantagonismus der geprüften Verbindungen deutlich stärker ausgeprägt ist als bei Procainamid.

Die therapeutische Breite als Quotient aus der letalen Dosis (LD 50) und der antiarrhythmisch wirkenden Dosis (ED 50 %) ist 4 mal (Beispiel 2) bzw. 2,8 mal (Beispiel 3) größer als beim Procainamid.

Tabelle 1: Antiarrhythmische Wirkung und akute Toxizität

| | Antiarrhythmische Wirkung 1) | | | | | Akute Toxizität | |
|---|---|---|---|---|---|---|---|
| | Wirksame Dosis | | Maximale Wirkung 4) | | | | |
| Verbindung | ED 20 % 2) | R.W. 3) | Dosis | △ % 5) | R.M.W. 6) | LD 50 mg/kg | Therapeutische Breite 7) |
| Beispiel 2 | 31,2 | 5,03 | 215 | 289 | 2,14 | 126 | 4,03 |
| Beispiel 3 | 32,7 | 4,80 | 215 | 233 | 1,73 | 90,5 | 2,76 |
| Procainamid | 157 | 1,00 | 681 | 135 | 1,00 | 227 | 1,00 |

1) Aconitinarrhythmie Ratte
2) Dosis (mg/kg) per os, welche die Aconitininfusionsdauer (min) um 50 [%] verlängert
3) R.W. = Relative Wirksamkeit; Procainamid = 1,00
4) Wirkung der höchsten nicht toxischen Dosis
5) Verlängerung der Aconitininfusionsdauer △ %
6) R.M.W. = Relative maximale Wirksamkeit
7) $\frac{\text{LD 50}}{\text{ED 50 \%}}$

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tab. tten, Filmtabletten, Dragees, Kapseln, Pillen, Pulver, Lösungen oder Suspensionen oder Depotformen.

Selbstverständlich kommen auch parenterale Zubereitungsformen, wie Injektionslösungen oder Zusätze zu Infusionslösungen, in Betracht. Weiterhin seien als Zubereitungen beispielsweise auch Suppositorien genannt.

Die entsprechenden Tabellen können beispielsweise durch Mischen des Wirkstoffs mit bekannten Hilfsstoffen, beispielsweise inerte Verdünnungsmittel, wie Dextrose, Zucker, Sorbit, Mannit, Polyvinylpyrrolidon, Calciumcarbonat, Calciumphosphat oder Milchzucker, Sprengmittel, wie Mais, Stärke, Alginsäure oder Polyvinylpyrrolidon, Bindemittel, wie Stärke oder Gelatine, Gleitmitteln, wie Magnesiumstearat oder Talkum und/oder Mitteln zur Erzielung des Depoteffektes, wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die

Drageehülle aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Saccharin, Cyclamat oder Zucker sowie beispielsweise Aromastoffe, wie Vanillin oder Orangenextrakt, enthalten. Sie können außerdem Suspendierhilfsstoffe, wie Natriumcarboxymethylcellulose, oder Konservierungsstoffe, wie Parahydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Geeignete Suppositorien lassen sich beispielsweise durch Vermischen mit dafür vorgesehenem Trägermaterial, wie Neutralfetten oder Polyäthylenglykolen bzw. dessen Derivaten, herstellen.

Die Einzeldosis einer erfindungsgemäßen Substanz am Menschen liegt bei 5 bis 100 mg, vorzugsweise bei 10 bis 80 mg.

Die in den folgenden Beispielen angebenen Teile sind Gewichtsteile. Sie verhalten sich zu den Volumenteilen wie Kilogramm zu Liter.

Beispiel 1

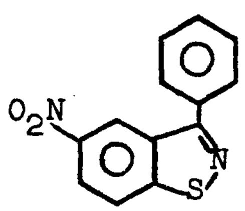

In einem Tantalautoklaven werden 26,15 Teile 2-Chlor-5-nitrobenzophenon, 3,2 Teile Schwefel in 300 Teilen Methanol mit 40 Teilen $NH_3$ bei einem Druck von 5 bar und einer Temperatur von 80°C 6 Stunden umgesetzt. Das Reaktionsgemisch wird auf 25°C abgekühlt, entspannt und der ausgefallene Feststoff abgesaugt. Die erhaltenen Kristalle werden mit Wasser chloridfrei gewaschen und getrocknet. Man erhält 24 Teile 3-Phenyl-5-nitro-1,2-benzisothiazol mit Fp 130°C. Die Ausbeute entspricht 94 % der Theorie.

Beispiel 2

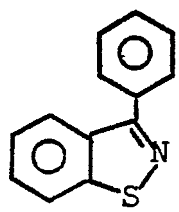

In einem Tantalautoklaven werden 216,5 Teile 2-Chlorbenzophenon, 32 Teile Schwefel und 100 Teile $NH_3$ in 1 000 Teilen Glykolmonomethyläther 6 Stunden bei 160°C umgesetzt. Nach Abkühlen und Entspannen wird das Reaktionsgemisch weitgehend vom Lösungsmittel befreit, mit 500 Teilen Wasser versetzt und mit 3 x 200 Teilen Methylenchlorid extrahiert. Die Methylenchloridlösung wird destillativ aufgearbeitet. Bei $Kp_1$ 150 bis 155°C werden 164 Teile 3-Phenyl-1,2-benzisothiazol mit Fp 70°C erhalten. Die Ausbeute entspricht 78 % der Theorie.

Beispiel 3

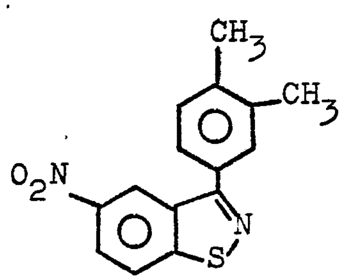

In einem Tantalautoklaven werden 145 Teile 2-Chlor-5-nitro-3',4'-dimethylbenzophenon, 16 Teile Schwefel und 100 Teile $NH_3$ in 800 Teilen Methanol 10 Stunden bei 80°C umgesetzt. Der Autoklaveninhalt wird abgesaugt und mit Wasser gewaschen. Man erhält 134 Teile 2-(3',4'-Dimethylphenyl)-5-nitro-1,2-benzisothiazol mit Fp 154°C. Die Ausbeute entspricht 94 % der Theorie.

Beispiel 4

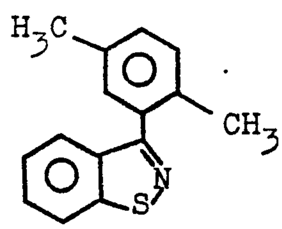

In einem Emaille-Autoklaven werden 245,5 Teile 2-Chlor-2',5'-dimethylbenzophenon, 32 Teile Schwefel und 100 Teile Ammoniak in 800 Teilen Methylglykol 6 Stunden bei 160°C umgesetzt. Nach Abkühlen und Entspannen wird das Lösungsmittel weitgehend abdestilliert, der Rückstand mit Wasser gewaschen und aus 1 000 Teilen Methanol umkristallisiert. Man erhält 192 Teile 3-(2',5'-Dimethylphenyl)-1,2-benzisothiazol mit Fp 87°C. Die Ausbeute entspricht 80 % der Theorie.

Beispiel 5

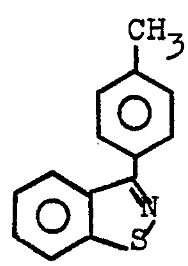

In einem Emailleautoklaven werden 230,5 Teile 2-Chlor-4'-methylbenzophenon, 32 Teile Schwefel und 100 Teile $NH_3$ in 800 Teilen Methylglykol 6 Stunden bei 160°C umgesetzt. Man erhält 200 Teile 3-(4'-Methylphenyl)-1,2-benzisothiazol

mit Fp 56°C. Die Ausbeute entspricht 89 % der Theorie.

In entsprechender Weise werden unter denselben Bedingungen und Molverhältnissen aus 2,5-Dichlor-4'-methylbenzophenon 5-Chlor-3-(4'-methylphenyl)-1,2-benzisothiazol vom Fp 121°C (85 % der Theorie; aus 2-Chlor-5-nitro-4'-methylbenzophenon 5-Nitro-3-(4'-methylphenyl)-1,2-benzisothiazol vom Fp 179°C (90 % der Theorie) hergestellt.

Beispiel 6

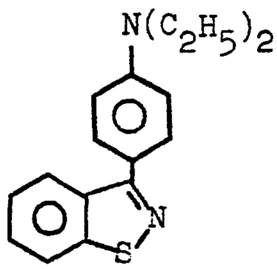

In einem Tantalautoklaven werden 28,75 Teile 2-Chlor-4'-diäthylamino-benzophenon, 3,2 Teile Schwefel und 50 Teile $NH_3$ in 400 Teilen Glykolmonoäthyläther 20 Stunden bei 160°C umgesetzt. Der Autoklavenaustrag wird vollständig eingeengt, der Rückstand in 500 Teilen Wasser aufgenommen und 3 mal mit 200 Teilen Methylenchlorid extrahiert. Die organische Phase wird getrocknet und eingeengt. Nach Umkristallisieren aus Methanol erhält man 20 Teile 3-(4'-Diäthylaminophenyl)-1,2-benzisothiazol mit Fp 85°C. Die Ausbeute entspricht 71 % der Theorie.

Beispiel 7

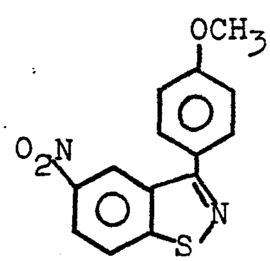

In einem Emailleautoklaven werden 291,5 Teile 2-Chlor-5-nitro-4'-methoxybenzophenon, 32 Teile Schwefel und 100 Teile Ammoniak in 1 000 Teilen Methanol 5 Stunden bei 80°C umgesetzt. Der Autoklaveninhalt wird abgesaugt, mit Wasser gewaschen und getrocknet. Man erhält 280 Teile 3-(4'-Methoxyphenyl)-5-nitro-1,2-benzisothiazol mit Fp 207°C. Die Ausbeute entspricht 97 % der Theorie.

Beispiel 8

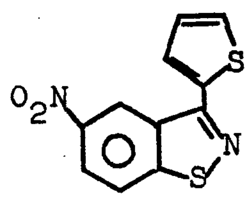

In einem Tantalautoklaven werden 26,7 Teile 2-(2'-Chlor-5'-nitrobenzoyl)-thiophen, 3,2 Teile Schwefel und 50 Teile $NH_3$ in 200 Teilen Methanol 10 Stunden bei 80°C umgesetzt. Der Autoklaveninhalt wird abgesaugt, mit Wasser gewaschen und aus Methylglykol umkristallisiert. Man erhält 21 Teile 5-Nitro-3-(thien-2'-yl)-1,2-benzisothiazol mit Fp 164°C. Die Ausbeute entspricht 80 % der Theorie.

Beispiel 9

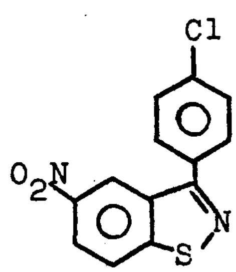

In einem Tantalautoklaven werden 29,6 Teile 2-Chlor-5-nitro-4'-chlorbenzophenon, 3,2 Teile Schwefel und 50 Teile $NH_3$ in 500 Teilen Methanol 24 Stunden bei 80°C umgesetzt. Der Autoklaveninhalt wird abgesaugt, mit Wasser gewaschen und getrocknet. Man erhält 26 Teile 3-(4'-Chlorphenyl)-5-nitro-1,2-benzisothiazol mit Fp 226°C. Die Ausbeute entspricht 89 % der Theorie.

Die vorliegende Erfindung wird durch die nachfolgenden Verwendungsbeispiele näher erläutert.

A. Beispiel für die Herstellung einer Verbindung der Formel Ib durch Chlorierung aus 1,2-Benzisothiazolen I.

3-(4-Chlormethylphenyl)-1,2-benzisothiazol

225 Teile 3-(4-Methylphenyl)-1,2-benzisothiazol werden in einer Rührapparatur auf 170°C erhitzt und unter Bestrahlung mit einer UV-Lampe werden innerhalb von 2 Stunden 100 Teile Chlor eingeleitet. Der Endpunkt der Reaktion wird gaschromatographisch bestimmt (Verschwinden des Ausgangsproduktes). Anschließend wird das Reaktionsgemisch abgekühlt, es wird abgesaugt und aus Methanol umkristallisiert. Man erhält 208 Teile 3-(4-Chlormethylphenyl)-1,2-benzisothiazol von einem Schmelzpunkt von 86 bis 89°C, was einer Ausbeute von 80 % der Theorie entspricht.

In entsprechender Weise wird unter denselben Bedingungen und Molverhältnissen 5-Chlor-3-(4'-chlormethylphenyl)-1,2-benzisothiazol mit Fp 116°C hergestellt.

B. Herstellung der Verbindungen IV.

Verwendungsbeispiel 1

3-[4'-(Imidazolin-(2")-yl)-phenyl]-1,2-benzisothiazol

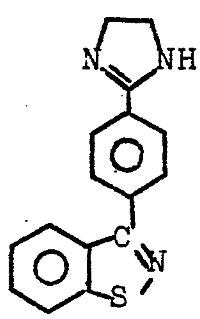

26 Teile 3-(4'-Chlormethylphenyl)-1,2-benzisothiazol, 6,4 Teile Schwefel und 300 Teile Toluol werden auf 50°C erwärmt und bei dieser Temperatur 12 Teile Äthylendiamin langsam zugegeben. Anschließend wird das Reaktionsgemisch 15 Stunden bei Rückflußtemperatur gerührt, heiß abfiltriert und das Filtrat auf 10 bis 15°C abgekühlt. Man erhält 21 Teile 3-(4'-Imidazolin-(2")-yl-phenyl)-1,2-benzisothiazol von einem Schmelzpunkt von 177°C. Das entspricht einer Ausbeute von 75 % der Theorie. Das Hydrochlorid der Verbindung hat einen Schmelzpunkt von 318°C.

Verwendungsbeispiel 2

3-[4'-Tetrahydropyrimidin-(2")-yl)-phenyl]-1,2-benzisothiazolhydrochlorid

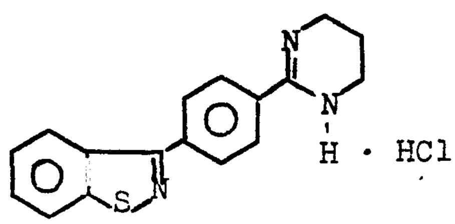

26 Teile 3-(4'-Chlormethylphenyl)-1,2-benzisothiazol, 6,4 Teile Schwefel und 300 Teile Toluol werden auf 50°C erwärmt. Bei dieser Temperatur werden 15 Teile 1,3-Diaminopropan langsam zugegeben. Das Reaktionsgemisch wird noch 20 Stunden bei Rückflußtemperatur gerührt. Anschließend werden innerhalb einer Stunde 20 Teile Chlorwasserstoffgas eingeleitet, es wird auf Raumtemperatur abgekühlt und der entstandene Feststoff abgesaugt. Nach dem Umkristallisieren aus Wasser, unter Zusatz von Aktivkohle, erhält man 18 Teile 3-[4'-(Tetrahydropyrimidin-(2")-yl)-phenyl]-1,2-benzisothiazolhydrochlorid von einem Schmelzpunkt von 314°C (Zersetzung). Die Ausbeute entspricht 54,6 % der Theorie.

Verwendungsbeispiel 3

3-[4'-(Methylimidazolin-(2")-yl)-phenyl]-1,2-benzisothiazol-hydrochlorid

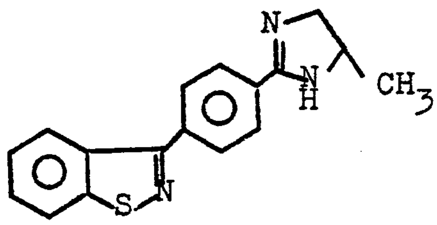
· HCl

26 Teile 3-(4'-Chlormethylphenyl)-1,2-benzisothiazol, 6,4 Teile Schwefel und 15 Teile 1,2-Diamonopropan werden in 400 Teilen Glykolmonomethyläther 15 Stunden auf 110°C erwärmt. Nach dem Abdestillieren des Lösungsmittels wird der Rückstand in 50 Teilen Methanol gelöst und unter Kühlen in 400 Teilen ätherische Salzsäure (15 Teile HCl in Diäthyläther) eingerührt. Die gebildeten Kristalle werden abgesaugt, in 300 Teilen Wasser gelöst, filtriert, das Filtrat mit conc. NaOH alkalisch gestellt und mit Methylenchlorid extrahiert. Die Methylenchloridphase wird getrocknet und 20 Teile Chlorwasserstoffgas eingeleitet. Man erhält 17 Teile des gewünschten Endstoffs, als farblose Kristalle, vom Schmelzpunkt 243°C. Die Ausbeute entspricht 52 % der Theorie.

Verwendungsbeispiel 4

5-Chlor-3-[4'-(imidazolin-(2")-yl)-phenyl]-1,2-benzisothiazol

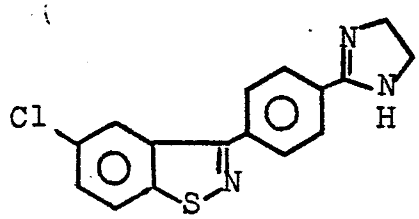

44 Teile 5-Chlor-3-(4'-chlormethylphenyl)-1,2-benzisothiazol, 9,6 Teile Schwefel und 18 Teile Äthylendiamin werden in 500 Teilen Toluol 20 Stunden am Rückfluß gekocht. Das Reaktionsgemisch wird heiß filtriert, das Filtrat abgekühlt und die gebildeten Kristalle abgesaugt. Nach Umkristallisieren aus Toluol unter Zusatz von Aktivkohle werden 26 Teile 5-Chlor-3-[4'-(imidazolin-(2")-yl)]-1,2-benzisothiazol vom Schmelzpunkt 195°C erhalten. Die Ausbeute entspricht 55 % der Theorie.

Verwendungsbeispiel 5

5-Chlor-3-[4'-(methylimidazolin-(2")-yl)-phenyl]-1,2-benzisothiazol-hydrochlorid

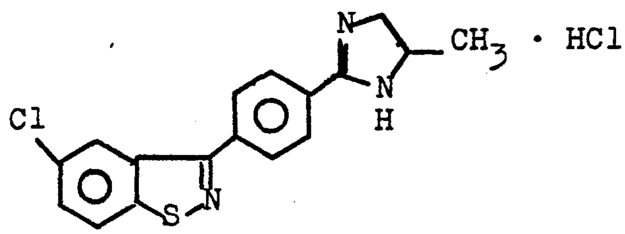

44 Teile 5-Chlor-3-(4'-chlormethylphenyl)-1,2-benzisothiazol, 9,6 Teile Schwefel und 22,5 Teile 1,2-Diaminopropan werden in 600 Teilen Toluol 24 Stunden am Rückfluß gekocht. Das Reaktionsgemisch wird in 500 Teilen Äther gelöst und 30 Teile Chlorwasserstoffgas unter Kühlen eingeleitet. Die gebildeten Kristalle werden abgesaugt und aus Wasser umkristallisiert. Man erhält 31 Teile 5-Chlor-3-[4'-(methylimidazolin-(2")-yl)-phenyl]-1,2-benzisothiazol vom Schmelzpunkt 248°C (Zersetzung). Die Ausbeute entspricht 57 % der Theorie.

Verwendungsbeispiele 6 - 9

Formulierungsbeispiele, die in üblicher Weise hergestellt werden:

6. Tabletten:

a)

| | |
|---|---|
| Ein Wirkstoff der Formel IV | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

b)

| | |
|---|---|
| Ein Wirkstoff der Formel IV | 20 mg |
| Lactose | 178 mg |
| Avicel | 80 mg |
| Polywachs 6000 | 20 mg |
| Magnesiumstearat | 2 mg |
| | 300 mg |

c)

| | |
|---|---|
| Ein Wirkstoff der Formel IV | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |
| | 280 mg |

Der Wirkstoff wird mit Polyvinylpyrrolidon in 10-prozentiger, wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4000), Hydroxypropylmethylcellulose, Talkum und Magnesiumstearat vermischt und zu Tabletten à 280 mg verpreßt.

7. Beispiel für Dragees:

| | |
|---|---|
| Ein Wirkstoff der Formel IV | 60 mg |
| Lactose | 90 mg |
| Maisstärke | 60 mg |
| Polyvinylpyrrolidon | 6 mg |
| Magnesiumstearat | 1 mg |
| | 217 mg |

Die Mischung der Wirkstoffsubstanz mit Lactose und Maisstärke wird mit einer 8-prozentigen, wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

8. Kapselformulierung:

| | |
|---|---|
| Ein Wirkstoff der Formel IV | 5,0 mg |
| Magnesiumstearat | 2,0 mg |
| Milchzucker | 19,3 mg |

9. Injektionslösung:

| | |
|---|---|
| Ein Wirkstoff der Formel IV | 10 mg |
| Natriumchlorid | 9 mg |
| destilliertes Wasser, q.s. auf auf 1,0 ml. | |

Patentansprüche

1. Verfahren zur Herstellung von 1,2-Benzisothiazolen der Formel

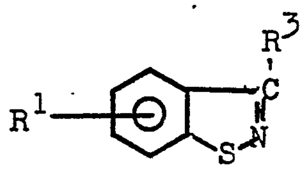 I,

worin $R^1$ Wasserstoff, einen aliphatischen, cycloaliphatischen oder einen, gegebenenfalls anellierten, aromatischen Rest, Halogen, eine Alkoxygruppe, Nitrogruppe oder den Rest 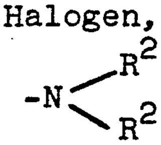, worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, und $R^3$ für einen aromatischen oder heterocyclischen Rest steht, dadurch gekennzeichnet, daß man o-Halogenarylketone der Formel

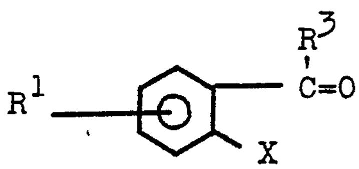 II,

worin $R^1$ und $R^3$ die vorgenannte Bedeutung besitzen und X für Halogen steht, mit Ammoniak und elementarem Schwefel umsetzt.

2. 1,2-Benzisothiazole der Formel

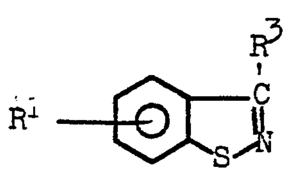 I,

worin $R^1$ Wasserstoff, einen aliphatischen, cycloaliphatischen oder einen gegebenenfalls anellierten, aromati-

schen Rest, Halogen, eine Alkoxygruppe, Nitrogruppe oder den Rest 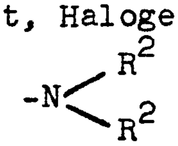 , worin die einzelnen Reste $R^2$ gleich oder verschieden sein können und jeweils Wasserstoff, einen aliphatischen, cycloaliphatischen, araliphatischen oder aromatischen Rest bedeuten, bezeichnet, und $R^3$ für einen substituierten einkernigen oder unsubstituierten mehrkernigen oder substituierten mehrkernigen, aromatischen Rest oder einen heterocyclischen Rest steht.

0000750

Europäisches Patentamt

# EUROPÄISCHER RECHERCHENBERICHT

Nummer der Anmeldung
EP 78 10 0536

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 503 699 (BASF AG)<br>* Patentanspruch *<br>-- | 1 |
| | CHEMICAL ABSTRACTS, Vol. 58,(1963) 11340c, 8 Ric. Sci Rend.Ser. B2 177-8 (1962)<br>* Zusammenfassung *<br>---- | 2 |

KLASSIFIKATION DER ANMELDUNG (Int.Cl.²)

C 07 D 275/04
C 07 D 417/04
// C 07 D 417/10
A 61 K 31/505
A 61 K 31/425

RECHERCHIERTE SACHGEBIETE (Int. Cl.²)

C 07 D 275/04
C 07 D 417/10
C 07 D 417/04
C 07 D 417/02

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 26-10-1978 | HENRY |